(19) **Europäisches Patentamt**
**European Patent Office**
**Office européen des brevets**

(11) **EP 1 515 657 B1**

(12) **EUROPÄISCHE PATENTSCHRIFT**

(45) Veröffentlichungstag und Bekanntmachung des Hinweises auf die Patenterteilung:
**19.03.2008   Patentblatt 2008/12**

(51) Int Cl.:
***A61B 17/86*** *(2006.01)*      ***A61F 2/44*** *(2006.01)*

(21) Anmeldenummer: **02737720.9**

(22) Anmeldetag: **26.06.2002**

(86) Internationale Anmeldenummer:
**PCT/CH2002/000347**

(87) Internationale Veröffentlichungsnummer:
**WO 2004/002344 (08.01.2004 Gazette 2004/02)**

(54) **KNOCHENFIXATIONSELEMENT**

BONE FIXING ELEMENT

ELEMENT DE FIXATION POUR OS

(84) Benannte Vertragsstaaten:
**AT BE CH CY DE DK ES FI FR GB GR IE IT LI LU MC NL PT SE TR**

(43) Veröffentlichungstag der Anmeldung:
**23.03.2005   Patentblatt 2005/12**

(73) Patentinhaber: **Synthes GmbH**
**4436 Oberdorf (CH)**

(72) Erfinder:
• **GOLDHAHN, Jörg**
**CH-8600 Dübendorf (CH)**

• **SEEBECK, Jörn**
**CH-8405 Winterthur (CH)**
• **SCHNEIDER, Erich**
**CH-7270 Davos (CH)**

(74) Vertreter: **Lusuardi, Werther**
**Dr. Lusuardi AG,**
**Kreuzbühlstrasse 8**
**8008 Zürich (CH)**

(56) Entgegenhaltungen:
**EP-A- 0 682 917          WO-A-01/26568**
**WO-A-02/38054          US-A1- 2001 049 559**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft ein Knochenfixationselement nach dem Oberbegriff des Anspruchs 1.

Bisherige Implantate berücksichtigen nur ungenügend die Reaktionen des umgebenden Gewebes, insbesondere des Knochens auf den Fremdkörper Implantat. Dabei wird vor allem die lokale Mikrozirkulation bzw. Perfusion geschädigt sowie die lokale Biomechanik verändert.

Insbesondere der Einfluss relativ steifer Implantate aus Titan oder Stahl ist bei bisherigen Implantaten nur ungenügend berücksichtigt worden. Um Ermüdungsbrüchen vorzubeugen, werden die Implantate so steif und fest als möglich konzipiert. Da das eingesetzte Implantat während der Frakturheilung und während der gesamten Zeit seines Verbleibs im Körper zumindest teilweise die Lastübertragung des Knochens übernimmt, kommt es reaktiv zur Ausdünnung der Knochenstruktur in den betroffenen Bereichen (Wolffsches Gesetz). Dieser Knochenumbau kann zur Auslockerung des Implantates führen.

Insbesondere Hohlzylinder, wie sie zur Frakturversorgung im osteoporotischen Knochen vorgesehen sind (z.B. gemäss DE-C 19628473 und DE-U 297 10 979) können zu diesen Erscheinungen führen. Dabei wäre gerade diese Kategorie von Implantaten für den Problemfall Osteoporose interessant, da man sich von diesen Implantaten eine geringere Häufigkeit von Implantatversagern verspricht. Der Knochen im Innern eines des Hohlzylinders soll erhalten bleiben und neuer Knochen durch Perforationen einwachsen. Beides sollte theoretisch zu einer besseren Verankerung des Implantates im osteoporotischen Knochen führen. Das Dokument DE-C 19 628 473 offenbart die im einleitenden Teil des Anspruchs 1 definierten Merkmale.

Hier will die Erfindung Abhilfe schaffen. Der Erfindung liegt die Aufgabe zugrunde, ein Knochenfixationsmittel zu schaffen, welches die oben erwähnten Nachteile vermeidet.

Die Schädigung der Mikrozirkulation und Perfusion beruht auf der Durchtrennung der versorgenden Blutgefässe während der Insertion des Implantates. Eigene Untersuchungen haben gezeigt, dass eine Erholung der Blutversorgung innerhalb und ausserhalb eines Hohlzylinders in sehr kurzer Zeit möglich ist. Für die Blutversorgung im Inneren des Hohlzylinders sind die Perforationen im Zylindermantel entscheidend sowie die zyklische Kompression, die eine Perfusion von Gewebeflüssigkeit zur Folge hat. Das Ausmass der Perfusion hängt wiederum von der Steifigkeit des Implantates ab. Die zyklische Kompression durch Belastung des Knochens im Heilungsverlauf führt ebenfalls zur Regeneration benachbarter Knochenabschnitte. Die knöcherne Integration eines Implantates hängt also entscheidend von seinen mechanischen Eigenschaften, insbesondere seiner Steifigkeit ab.

Es ist Aufgabe der Erfindung, ein Knochenfixationsmittel zu schaffen, das dank seiner Konstruktion die Forderung nach adaptierter Steifigkeit erfüllt und damit eine Verankerung im Knochen, insbesondere im osteoporotischen Knochen, langfristig gewährleistet und gleichzeitig den biologischen Rahmenbedingungen gerecht wird. Es sichert den Erhalt verbliebener knöcherner Strukturen und fördert das Ein- und Anwachsen von neuem Knochen.

Die Erfindung löst die gestellte Aufgabe mit einem Knochenfixationsmittel, welches die Merkmale des Anspruchs 1 aufweist.

Die durch die Erfindung erreichten Vorteile sind im wesentlichen darin zu sehen, dass dank des erfindungsgemässen Knochenfixationsmittels die Steifigkeit des Hohlzylinders derjenigen des ihn umgebenden Knochens weitestgehend angenähert werden kann, insbesondere den in einem Wirbelkörper vorhandenen Unterschieden zwischen Kortikalis (E-Modul von ca. 10'000 bis 20'000 MPa) und Spongiosa (E-Modul von ca. 100 bis 5'000 MPa). Ausserdem fällt unter Belastung die Auslenkung sowie Kompression des Knochenfixationsmittels im Innern des Wirbelkörpers stärker aus als im Randbereich.

Diese Steifigkeitsänderung über die Längsachse des Implantates kann mit zwei Massnahmen erreicht werden:

 1. Reduktion der Wandstärke im Bereich des Übergangs zwischen Kortikalis und Spongiosa; und
 2. durch einen Porositätsgradienten, der in Richtung des offenes Endes des Hohlzylinders zunimmt.

Das axiale und polare Flächenträgheitsmoment ($I_x$ und $I_p$) als Messgrösse der Steifigkeit hängen bei dünnwandigen Kreisprofilen mit dem mittleren Radius $R_m$ überschlägig folgendermassen von der Wandstärke t und Porosität p (Porenfläche/Gesamtoberfläche) ab.

$$I_p \approx 2 \cdot \pi \cdot R_m{}^3 \cdot t \cdot p$$

$$I_x \approx \pi \cdot R_m{}^3 \cdot t \cdot p$$

Die maximal erreichbare Porosität bei dichtmöglichster Anordnung von kreisförmigen Poren gleicher Grösse beträgt in etwa 90%. Um die strukturelle Integrität der Wandung zu gewährleisten sollte sie aber 85% nicht überschreiten.

Durch eine parametrische Finite Elemente Analyse konnte ermittelt werden, das die Steifigkeit eines Kreisquerschnittes exponentiell von der Wandstärke abhängt.

$$W_{querschnitt} \sim t^{2,7343}$$

gilt für $t/R_m = \{0.4 \ldots 0.08\}$

d.h. eine Reduktion der Wandstärke um 50% führt zu einer Halbierung der Flächenträgheitsmomente und reduziert die Steifigkeit des Kreisquerschnittes auf etwa 15%.

Bei einer besonderen Ausführungsform des Knochenfixationselementes nimmt dessen Steifigkeit vom hinteren zum vorderen Ende kontinuierlich ab. Vorzugsweise ist die Steifigkeit des Hohlzylinders im vorderen Drittel des Hohlzylinders um mindestens 20 % kleiner ist, als diejenige im hinteren Drittel des Hohlzylinders.

Bei einer besonderen Ausführungsform ist das hintere Ende des Knochenfixationselementes mit Mitteln versehen ist, welche zur form- oder kraftschlüssigen Aufnahme eines Kraftträgers, vorzugsweise einer Knochenplatte geeignet sind.

Bei einer besonderen Ausführungsform nimmt die Porosität - als Verhältnis S/M zwischen der Summe S der n Perforationsflächen zur gesamten Mantelfläche M - vom hinteren zum vorderen Ende hin zu. Vorzugsweise ist die Porosität im vorderen Drittel des Hohlzylinders um mindestens 20 % grösser ist, als diejenige im hinteren Drittel des Hohlzylinders.

Bei einer weiteren Ausführungsform nimmt die Wandstärke des Hohlzylinders vom hinteren zum vorderen Ende hin vorzugsweise kontinuierlich ab. Vorzugsweise ist die Wandstärke des Hohlzylinders im vorderen Drittel des Hohlzylinders um mindestens 20 % kleiner ist, als diejenige im hinteren Drittel des Hohlzylinders.

Bei einer besonderen Ausführungsform weisen die n Perforationen in der Mantelfläche des Hohlzylinders mindesten 0,5 mm als kleinsten Durchmesser auf.

Vorzugsweise ist ein Aussengewinde mindestens teilweise auf der Mantelfläche des Hohlzylinders angebracht.

Die Form der Perforationen kann rund oder oval aber auch eckig, vorzugsweise dreieckig oder viereckig sein.

Bei einer besonderen Ausführungsform nimmt die Anzahl der Perforationen pro Zehntel der Zylinderhöhe zum vorderen Ende hin zu, wobei aber die Fläche der einzelnen Perforationen konstant bleibt. Bei einer anderen Ausführungsform bleibt die Anzahl der Perforationen pro Zehntel der Zylinderhöhe zum vorderen Ende hin konstant, wobei aber die Fläche der einzelnen Perforationen gegen das vordere Ende hin zunimmt.

Die Erfindung und Weiterbildungen der Erfindung werden im folgenden anhand der teilweise schematischen Darstellungen mehrerer Ausführungsbeispiele noch näher erläutert.

Es zeigen:

Fig. 1 eine Teil-Abwicklung der Hohlzylinderwand mit zunehmender Porosität durch Vergrösserung der Perforationen mit einer Anzahl von Querschnitten durch die Hohlzylinderwand;

Fig. 2 eine Variante zur Veränderung der Porosität der Hohlzylinderwand mit ovalen Perforationen;

Fig. 3 eine Variante zur Veränderung der Porosität der Hohlzylinderwand mit quadratischen Perforationen;

Fig. 4 eine Variante zur Veränderung der Porosität der Hohlzylinderwand mit dreieckigen Perforationen; und

Fig. 5 eine perspektivische Darstellung eines teilweise aufgeschnittenen Knochenfixationselementes.

Die nachfolgende Beschreibung bevorzugter Ausführungsformen dient im Zusammenhang mit den Zeichnungen dem besseren Verständnis der zu Grunde liegenden Erfindung.

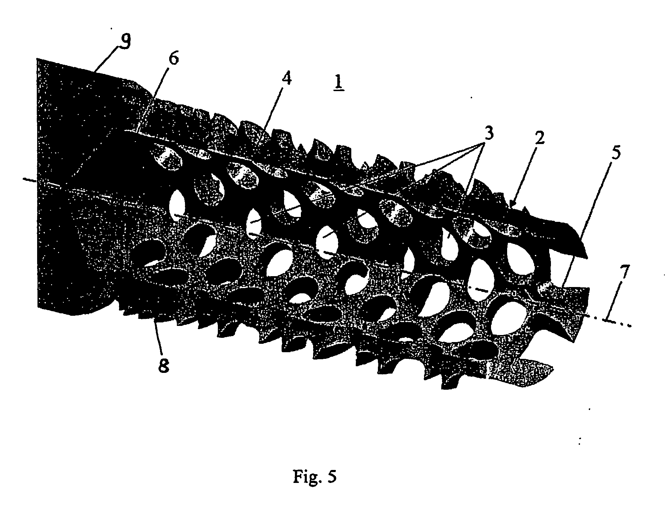

Das in Fig. 5 dargestellte Knochenfixationselement 1 besteht im wesentlichen aus einem Hohlzylinder 2 mit einer grösseren Anzahl von Perforationen 3 in dessen Mantelfläche 4, einem vorderen, zur Einführung in den Knochen geeigneten, offenen Ende 5, einem hinteren Ende 6 und einer Zylinderlängsachse 7.

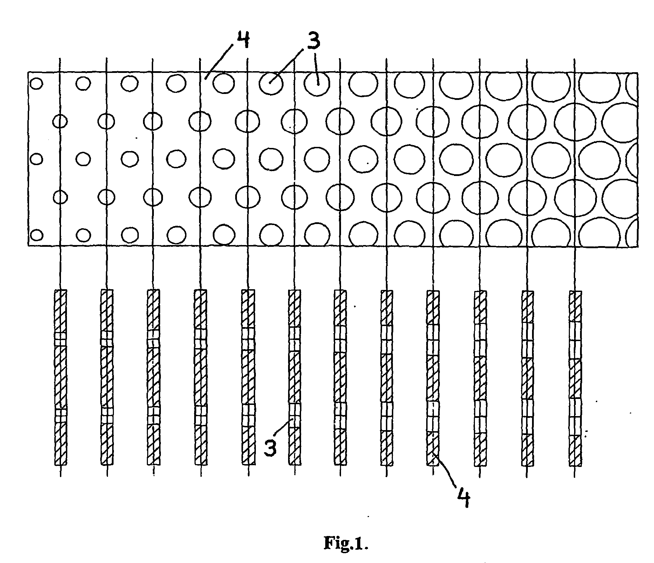

Die in Fig. 1 dargestellte Teil-Abwicklung der Hohlzylinderwand zeigt wie sich die Poren 3 hinten nach vorne kontinuierlich vergrössern, so dass die Steifigkeit des Hohlzylinders über seine Längsachse vom hinteren Ende zum vorderen Ende hin entsprechend abnimmt.

Am hinteren Ende 6 ist die Mantelfläche 4 mit einem Aussengewinde 8 versehen. Den hinteren Abschluss des Knochenfixationselementes bildet ein konischer Kopf 9, der in eine (zeichnerisch nicht dargestellte Knochenplatte) einsetzbar ist.

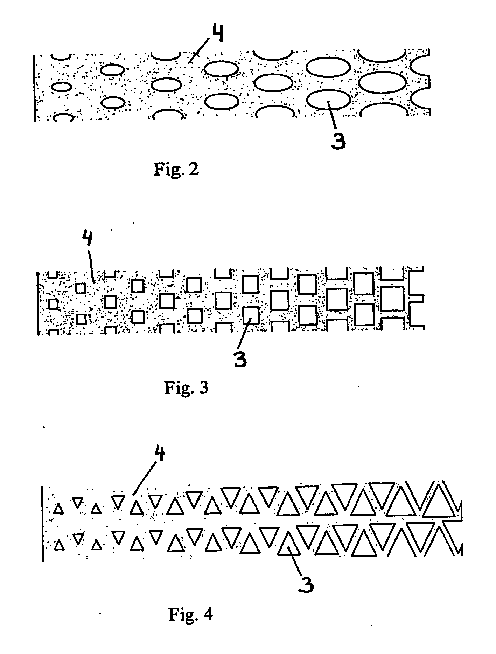

In den Fig. 2 bis 4 sind Varianten dargestellt zur Veränderung der Porosität mit veränderten Lochgeometrien. Statt kreisförmiger Perforationen werden ovale, quadratische oder dreieckige Perforationen verwendet, wobei sich auch diese von hinten nach vorne regelmässig vergrössern. Der Zweck dieser Lochgeometrien besteht darin, die Steifigkeit des Knochenfixationselementes von hinten nach vorne abnehmen zu lassen. Dies könnte auch dadurch erreicht werden, dass gleich grosse Löcher verwendet werden, welche aber von hinten nach vorne häufiger oder dichter beieinander auftreten.

**Patentansprüche**

1. Knochenfixationselement (1) in Form eines Hohlzylinders (2) mit n Perforationen (3) in dessen Mantelfläche (4), einem vorderen, zur Einführung in den Knochen geeigneten, offenen Ende (5), einem hinteren Ende (6) und einer Zylinderlängsachse (7) **dadurch gekennzeichnet, dass**

die Steifigkeit des Hohlzylinders (2) über seine Längsachse (7) vom hinteren Ende (6) zum vorderen Ende (5) hin abnimmt.

2. Knochenfixationselement (1) nach Anspruch 1, **dadurch gekennzeichnet, dass** die Steifigkeit kontinuierlich abnimmt.

3. Knochenfixationselement (1) nach Anspruch 1 oder 2, **dadurch gekennzeichnet, dass** die Steifigkeit des Hohlzylinders (2) im vorderen Drittel des Hohlzylinders (2) um mindestens 20 % kleiner ist, als diejenige im hinteren Drittel des Hohlzylinders (2).

4. Knochenfixationselement (1) nach einem der Ansprüche 1 bis 3, **dadurch gekennzeichnet, dass** das hintere Ende (6) mit Mitteln versehen ist, welche zur form- oder kraftschlüssigen Aufnahme eines Kraftträgers, vorzugsweise einer Knochenplatte geeignet sind.

5. Knochenfixationselement (1) nach einem der Ansprüche 1 bis 4, **dadurch gekennzeichnet, dass** die Porosität - als Verhältnis S/M zwischen der Summe S der n Perforationsflächen zur gesamten Mantelfläche (4) M - vom hinteren Ende (6) zum vorderen Ende (5) hin zunimmt.

6. Knochenfixationselement (1) nach Anspruch 5, **dadurch gekennzeichnet, dass** die Porosität im vorderen Drittel des Hohlzylinders (2) um mindestens 20 % grösser ist, als diejenige im hinteren Drittel des Hohlzylinders (2).

7. Knochenfixationselement (1) nach einem der Ansprüche 1 bis 6, **dadurch gekennzeichnet, dass** die Wandstärke des Hohlzylinders (2) vom hinteren Ende (6) zum vorderen Ende (5) hin vorzugsweise kontinuierlich abnimmt.

8. Knochenfixationselement (1) nach Anspruch 7, **dadurch gekennzeichnet, dass** die Wandstärke des Hohlzylinders (2) im vorderen Drittel des Hohlzylinders (2) um mindestens 20 % kleiner ist, als diejenige im hinteren Drittel des Hohlzylinders (2).

9. Knochenfixationselement (1) nach einem der Ansprüche 1 bis 8, **dadurch gekennzeichnet, dass** die n Perforationen (3) in der Mantelfläche (4) des Hohlzylinders (2) mindesten 0,5 mm als kleinsten Durchmesser aufweisen.

10. Knochenfixationselement (1) nach einem der Ansprüche 1 bis 9, **dadurch gekennzeichnet, dass** ein Aussengewinde (8) mindestens teilweise auf der Mantelfläche (4) des Hohlzylinders (2) angebracht ist.

11. Knochenfixationselement (1) nach einem der Ansprüche 1 bis 10, **dadurch gekennzeichnet, dass** die Form der Perforationen (3) rund oder oval ist.

12. Knochenfixationselement (1) nach einem der Ansprüche 1 bis 10, **dadurch gekennzeichnet, dass** die Form der Perforationen eckig, vorzugsweise dreieckig oder viereckig ist.

13. Knochenfixationselement (1) nach einem der Ansprüche 1 bis 12, **dadurch gekennzeichnet, dass** die Anzahl der Perforationen (3) pro Zehntel der Zylinderhöhe zum vorderen Ende (5) hin zunimmt aber die Fläche der einzelnen Perforationen (3) konstant bleibt.

14. Knochenfixationselement (1) nach einem der Ansprüche 1 bis 13, **dadurch gekennzeichnet, dass** die Anzahl der Perforationen (3) pro Zehntel der Zylinderhöhe zum vorderen Ende (5) hin konstant bleibt aber die Fläche der einzelnen Perforationen (3) gegen das vordere Ende (5) hin zunimmt.

## Claims

1. Bone-fixation element (1) in the form of a hollow cylinder (2) with n perforations (3) in the circumferential surface (4) of the hollow cylinder (2), an open front end (5), suitable for introduction into the bone, a rear end (6) and a longitudinal axis (7) of the cylinder, **characterized in that** the stiffness of the hollow cylinder (2) decreases along its longitudinal axis (7) from the rear end (6) to the front end (5).

2. The bone-fixation element (1) of claim 1, **characterized in that** the stiffness decreases continuously.

3. The bone-fixation element (1) of claims 1 or 2, **characterized in that** the stiffness of the hollow cylinder (2) in the front third of the hollow cylinder (2) is at least 20% lower than that in the rear third of the hollow cylinder (2).

4. The bone-fixation element (1) of one of the claims 1 to 3, **characterized in that** the rear end (6) is provided with means, which are suitable for accommodating a force carrier, preferably a bone plate, positively or non-positively.

5. The bone-fixation element (1) of one of the claims 1 to 4, **characterized in that** the porosity, as the ratio of the sum S of the n perforation areas to the total circumferential area (4) M, increases from the rear end (6) to the front end (5).

6. The bone-fixation element (1) of claim 5, **characterized in that** the porosity in the front third of the hollow cylinder (2) is greater than that in the rear third of the hollow cylinder (2) by at least 20%.

7. The bone fixation element (1) of one of the claims 1 to 6, **characterized in that** the wall thickness of the hollow cylinder (2) decreases, preferably continuously, from the rear end (6) to the front end (5)

8. The bone fixation element (1) of claim 7, **characterized in that** the wall thickness of the hollow cylinder (2) in the front third of the hollow cylinder (2) is at least 20% less than that in the rear third of the hollow cylinder (2).

9. The bone fixation element (1) of one of the claims 1 to 8, **characterized in that** the smallest diameter of the n perforations (3) in the circumferential surface (4) of the hollow cylinder (2) is at least 0.5 mm.

10. The bone fixation element (1) of one of the claims 1 to 9, **characterized in that** an external thread (8) is provided at least partially on the circumferential surface (4) of the hollow cylinder (2).

11. The bone fixation element (1) of one of the claims 1 to 10, **characterized in that** the shape of the perforations (3) is round or oval.

12. The been fixation element (1) of one of the claims 1 to 10, **characterized in that** the shape of the perforations is angular, preferably triangular or quadrilateral.

13. The bone fixation element (1) of one of the claims 1 to 12, **characterized in that** the number of perforations (3) per tenth of the height of the cylinder increases in the direction of the front end (5), while the area of the individual perforations (3) remains constant.

14. The bone fixation element (1) of one of the claims 1 to 13, **characterized in that** the number of perforations (3) per tenth of the cylinder height remains constant in the direction of the front end (5), but that the area of the individual perforations (3) in the direction of the front end (5) increases.

**Revendications**

1. Élément de fixation osseuse (1) sous la forme d'un cylindre creux (2) présentant n perforations (3) sur son enveloppe externe (4), une extrémité avant (5) ouverte appropriée pour l'insertion dans l'os, une extrémité arrière (6) et un axe longitudinal de cylindre (7) **caractérisé en ce que** la rigidité du cylindre creux (2) diminue sur son axe longitudinal (7) de l'extrémité arrière (6) vers l'extrémité avant (5).

2. Élément de fixation osseuse (1) selon la revendication 1, **caractérisé en ce que** la rigidité diminue progressivement.

3. Élément de fixation osseuse (1) selon la revendication 1 ou 2, **caractérisé en ce que** la rigidité du cylindre creux (2) dans le tiers avant du cylindre creux (2) est inférieure d'au moins 20% à celle dans le tiers arrière du cylindre creux (2).

4. Élément de fixation osseuse (1) selon l'une quelconque des revendications 1 à 3, **caractérisé en ce que** l'extrémité arrière (6) est pourvue de moyens qui sont appropriés pour loger par emboîtement ou par enfoncement forcé un dispositif porteur, de préférence une plaque d'ostéosynthèse.

5. Élément de fixation osseuse (1) selon l'une quelconque des revendications 1 à 4, **caractérisé en ce que** la porosité, exprimée en tant que rapport S/M de la somme S des n surfaces de perforation à l'ensemble de l'enveloppe externe (4) M augmente de l'extrémité arrière (6) vers l'extrémité avant (5).

6. Élément de fixation osseuse (1) selon la revendication 5, **caractérisé en ce que** la porosité dans le tiers avant du cylindre creux (2) est supérieure d'au moins 20% à celle dans le tiers arrière du cylindre creux (2).

7. Élément de fixation osseuse (1) selon l'une quelconque des revendications 1 à 6, **caractérisé en ce que** l'épaisseur de paroi du cylindre creux (2) rétrécit de préférence progressivement de l'extrémité arrière (6) vers l'extrémité avant (5).

8. Élément de fixation osseuse (1) selon la revendication 7, **caractérisé en ce que** l'épaisseur de paroi du cylindre creux (2) dans le tiers avant du cylindre creux (2) est inférieure d'au moins 20% à celle dans le tiers arrière du cylindre creux (2).

9. Élément de fixation osseuse (1) selon l'une quelconque des revendications 1 à 8, **caractérisé en ce que** le plus petit diamètre des n perforations (3) dans l'enveloppe externe (4) du cylindre creux (2) est d'au moins 0,5 mm.

10. Élément de fixation osseuse (1) selon l'une quelconque des revendications 1 à 9, **caractérisé en ce qu'**un filetage (8) est prévu au moins en partie sur l'enveloppe externe (4) du cylindre creux (2).

11. Élément de fixation osseuse (1) selon l'une quelconque des revendications 1 à 10, **caractérisé en ce**

**que** la forme des perforations (3) est ronde ou ovale.

12. Élément de fixation osseuse (1) selon l'une quelconque des revendications 1 à 10, **caractérisé en ce que** la forme des perforations est angulaire, de préférence triangulaire ou quadrangulaire.

13. Élément de fixation osseuse (1) selon l'une quelconque des revendications 1 à 12, **caractérisé en ce que** le nombre de perforations (3) par dixième de la hauteur du cylindre augmente dans la direction de l'extrémité avant (5) mais la surface des perforations individuelles (3) reste constante.

14. Élément de fixation osseuse (1) selon l'une quelconque des revendications 1 à 13, **caractérisé en ce que** le nombre de perforations (3) par dixième de la hauteur du cylindre reste constante dans la direction de l'extrémité avant (5) mais la surface des perforations individuelles (3) augmente dans la direction de l'extrémité avant (5).

**Fig.1.**

Fig. 2

Fig. 3

Fig. 4

8

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19628473 C
- DE 29710979 U